# EUROPEAN PATENT APPLICATION

(11) **EP 2 008 578 A1**
(43) Date of publication of application: **31.12.2008**
(21) Application number: 07740579.3
(22) Date of filing: 30.03.2007
(51) Int. Cl.: A61B 5/00, G06Q 50/00

(54) **MEDICAL IMAGE DISPLAY DEVICE AND PROGRAM**

(30) Priority: 14.04.2006 JP 2006111901
(71) Applicant: Konica Minolta Medical & Graphic, Inc., Hino-shi, Tokyo 191-8511 (JP)
(72) Inventor: CHEN, Defeng, 1, Sakura-machi, Hino-shi, Tokyo 191-8511 (JP)
(74) Representative: Henkel, Feiler & Hänzel
(86) International application number: PCT/JP2007/057143
(87) International publication number: WO 2007/119615

(57) **Abstract**

A medical image display apparatus is provided with improvement in operability in adding and correcting annotations and convenience for use. An annotation registration table (74) for storing modality information, testing portion information, and annotation, which are correspondingly associated to each other, is prepared in advance in a memory (70). A CPU (10) of a testing-image device (1) stores medical image data received from a modality (M) in a testing-image waiting list table (72). The CPU (10) extracts the modality information and the testing portion information from header information that the medical image data designated by an operation of an input unit (20) contain. The CPU (10) reads out annotation data consistent with the modality information and the testing portion information from the annotation registration table (74). The CPU (10) displays the read-out annotation data on a display unit (30) and updates the medical image data by attaching the selected annotation data to a place on coordinates designated on a medical image.

## Description

### Technical Field

The present invention relates to a medical image display apparatus and a program for displaying a medical image based on medical image data.

### Background Art

Various medical image generating apparatuses (hereinafter referred to as "modality") such as a computed tomography (CT) apparatus, a computed radiography (CR) apparatus, a magnetic resonance imaging (MRI) apparatus, a mammographic apparatus, and an ultrasonic diagnostic apparatus, for taking a picture of a human body to generate medical image data including a subject image, tend to be integrated into a wide variety of systems.

Specifically, order information including patient information and testing information is transmitted from a hospital information system (HIS) or a radiological information system (RIS), and the modality takes photographs based on the order information in accordance with operations by a photographic operator. Medical image data including the image obtained by the photographing is generated in a data format based on, for example, a digital imaging and communications in medicine (DICOM) standard, and then the medical image data is transferred to a picture archiving and communication system (PACS).

Before the medical image data is transferred, the photographic operator needs to check and confirm whether or not the photographing is carried out based on the order information, whether or not the image data has image quality suitable for diagnosis, and whether or not the medical image data has various pieces of accurate information. In view of such circumstances, it is hoped that an image testing apparatus which confirms and corrects the medical image data (hereinafter an act of confirming and correcting the medical image data is referred to as "image testing") before transferring the medical image data, is put to practical use.

The image testing apparatus is a medical image display apparatus that receives the order information transmitted from the HIS or the RIS and the medical image data transmitted from the modality to display the order information and the medical image data. The photographic operator visually checks the order information and the medical image data to correct and adjust the data using the image testing apparatus.

Since the photographing using the modality requires a lot of testing conditions such as a region to be examined, posture, and a photographing direction of a patient to be photographed, it is difficult to determine the testing conditions only from the medical image on the screen. Therefore, a technique for recording an image indicating an annotation (hereinafter referred to as "annotation image") such as a testing condition in photographing as a part of the medical image using a lead marker, has been employed. With respect to the record of the annotation image, a technique for storing a plurality of pieces of image data of the annotations in advance in order to synthesize a selected image data and the medical image data is also known (see Patent Document 1) in addition to the physical technique using the lead marker.
Patent Document 1: Japanese Patent Application Laid-Open Publication No. 2005-270421

### Disclosure of Invention

### Problem to be Solved by the Invention

The technique disclosed in Patent Document 1, however, has the following drawbacks in correcting the annotation image synthesized by a modality such as an ultrasonic diagnostic apparatus or in newly adding the annotation image.

Because the annotation image depends on an examined region, a photographing direction, customary practices and specifications of each user, and modalities, the number of kinds of the annotation images becomes as large as several hundreds or more. Therefore, in cases where the medical image data is processed to add or correct the annotation image, it is necessary to select a desired annotation from the huge number of annotations, thereby requiring extremely troublesome operations.

In view of the foregoing, it is an object of the present invention to improve operability in adding or correcting an annotation and to realize an user-friendly medical image display apparatus.

### Means for Solving the Problem

In order to solve the above-describedproblem, according to a first aspect of the present invention, a medical image display apparatus includes: a storage section to store annotation data in a manner associated with a medical image generating apparatus for each medical image generating apparatus; a display section to display a medical image based on medical image data received from the medical image generating apparatus; a list display section to read out annotation data from the storage section, the annotation data being associated with the medical image generating apparatus from which the medical image data is received, and to display a list of the annotation data; a selection section to select annotation data from the list displayed by the list display section in accordance with an operation by a user; and a synthesizing section to synthesize the received medical image data and the annotation data selected by the selection section.

Preferably, the medical image display apparatus further includes an obtaining section to obtain a testing condition in the medical image generating apparatus, wherein the storage section includes a testing condition storage section to store annotation data in a manner associated with each testing condition, and the list display sectionmay read out annotation data from the testing condition storage section, the annotation data being associated with the testing condition obtained by the obtaining section, and displays a list of the annotation data.

According to a second aspect of the present invention, amedical image display apparatus includes : a testing condition storage section to store annotation data in a manner associated with a testing condition in a medical image generating apparatus for each testing condition; a display section to display a medical image based on medical image data received from the medical image generating apparatus; an obtaining section to obtain a testing condition in the medical image generating apparatus from which the medical image data is received; a list display section to read out annotation data from the testing condition storage section, the annotation data being associated with the testing condition obtained by the obtaining section, and to display a list of the annotation data; a selection section to select annotation data from the list displayed by the list display section in accordance with anoperationbyauser; and a synthesizing section to synthesize the received medical image data and the annotation data selected by the selection section.

Preferably, the medical image data contains a testing condition in the medical image generating apparatus, the obtaining section includes an extraction section to extract the testing condition from the received medical image data, and the list display section reads out annotation data from the testing condition storage section, the annotation data being associated with the testing condition extracted by the extraction section, and displays a list of the annotation data.

Preferably, the obtaining section includes a reception section to receive a testing condition in the medical image generating apparatus from a medical information system which orders generation of the medical image data, and the list display section reads out annotation data from the testing condition storage section, the annotation data being associated with the testing condition received by the reception section, and displays a list of the annotation data.

Preferably, the medical image display apparatus further includes a designation section to designate an annotation display position on the medical image displayed by the display section, wherein the synthesizing section synthesizes the annotation data selected by the selection section at the position designated by the designation section on the medical image, and updates the medical image data.

According to a third aspect of the present invention, a program causing a computer to function as: a storage section to store annotation data in a manner associated with a medical image generating apparatus for each medical image generating apparatus; a display section to display a medical image based on medical image data received from the medical image generating apparatus; a list display section to read out annotation data from the storage section, the annotation data being associated with the medical image generating apparatus from which the medical image data is received, and to display a list of the annotation data; a selection section to select annotation data from the list displayed by the list display section in accordance with an operation by a user; and a synthesizing section to synthesize the received medical image data and the annotation data selected by the selection section.

According to a forth aspect of the present invention, a program causing a computer to function as: a testing condition storage section to store annotation data in a manner associated with a testing condition in a medical image generating apparatus for each testing condition; a display section to display a medical image based on medical image data received from the medical image generating apparatus; an obtaining section to obtain a testing condition in the medical image generating apparatus from which the medical image data is received; a list display section to read out annotation data from the testing condition storage section, the annotation data being associated with the testing condition obtained by the obtaining section, and to display a list of the annotation data; a selection section to select annotation data from the list displayed by the list display section in accordance with anoperationbyauser; and a synthesizing section to synthesize the received medical image data and the annotation data selected by the selection section.

### Effect of the Invention

According to a first or third aspect of the present invention, annotation data, which is associated with a medical image generating apparatus from which medical image data is received, is read out to display a list of the annotation data. Therefore, a user can easily select desired annotation data from the list of the annotation data for each medical image generating apparatus in order to synthesize the selected annotation data on the medical image data. Accordingly, it is possible to improve operability in adding or correcting an annotation and to realize an user-friendly medical image display apparatus.

According to a second or fourth aspect of the present invention, a testing condition in a medical image generating apparatus is obtained, and annotation data which is associated with the testing condition is read out to display a list of the annotation data. Therefore, a user can easily select desired annotation data from the list of the annotation data for each testing condition to synthesize the selected annotation data on the medical image data. Accordingly, it is possible to improve operability in adding or correcting an annotation and to realize an user-friendly medical image display apparatus.

In the case of extracting the testing condition from the medical image data, the list of the annotation data suitable for the medical image data can be displayed.

In the case of receiving the testing condition from a medical information system, the list of the annotation data suitable for order for generation of the medical image data can be displayed.

In the case of synthesizing the selected annotation data at the designated position on the displayed medical image to update the medical image data, a user can record the annotation data at the desired position. Therefore, it is possible to add a new annotation to the medical image data and to correct an annotation recorded on the medical image data.

### Brief Description of Drawings

FIG. 1 is a block diagram showing an example of a configuration of a radiological department system.
FIG. 2A shows an example of a data structure of medical image data.
FIG. 2B shows an example of a data structure of header information.
FIG. 3 is a block diagram showing an example of a functional configuration of an image testing apparatus.
FIG. 4 shows an example of a data structure of an annotation registration table according to a first embodiment.
FIG. 5 is a flowchart for explaining a specific operation of the image testing apparatus.
FIG. 6 is a flowchart for explaining an annotation correction processing according to the first embodiment.
FIG. 7A shows a display example on a screen of the image testing apparatus.
FIG. 7B shows a display example on a screen of the image testing apparatus.
FIG. 8 shows an example of a data structure of order information.
FIG. 9 shows an example of a data structure of an annotation registration table according to a second embodiment.
FIG. 10 is a flowchart for explaining an annotation correction processing according to the second embodiment.

### Best Mode for Carrying Out the Invention

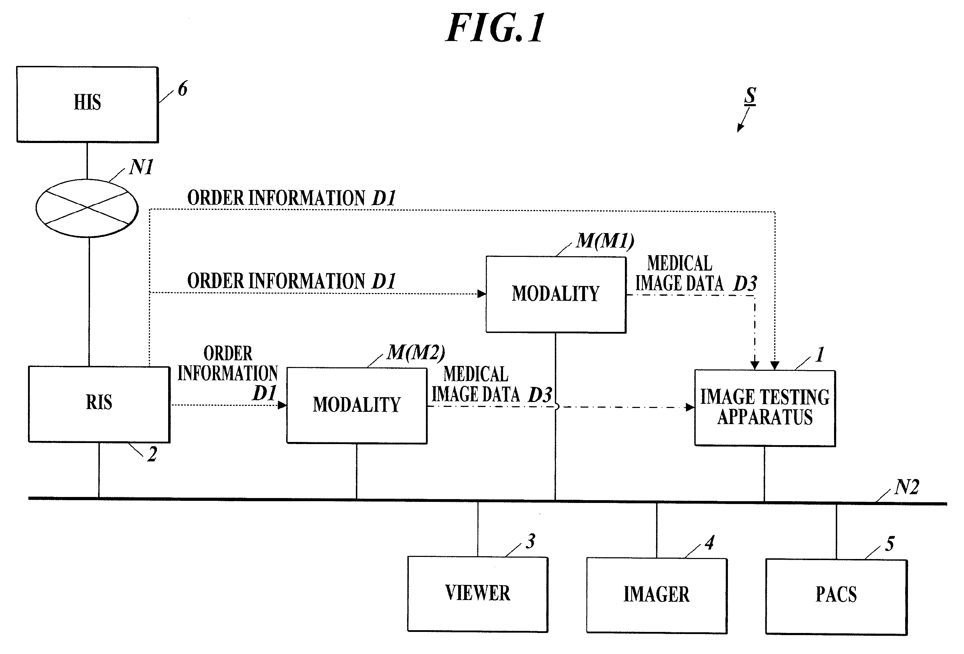

Embodiments in which a medical image display apparatus of the present invention is applied to an image testing apparatus 1 of FIG. 1 will be described below with reference to FIGS. 1 to 10.

An outline of a radiological department system S having the image testing apparatus 1 will be described with reference to FIG. 1. FIG. 1 is a block diagram showing an example of a configuration of a radiological department system S. As shown in FIG. 1, in the radiological department system S, the image testing apparatus 1, a RIS 2, modalities M (M1 and M2), a viewer 3, an imager 4, and a PACS 5 are connected to one another through a communication network N2 to achieve data communication with one another. The RIS 2 can conduct data communication with a HIS 6 through a hospital network N1.

The HIS (Hospital Information System) 6 includes a computer which has a CPU (Central Processing Unit), a storage section, an input section, a display section, and a communication section. The HIS 6 totally manages hospital information such as medical accounting, a medical appointment, management of electronic medical charts, and orders fortesting or medicine to each department. The HIS 6 accepts a request for photography in the radiological department system S from a doctor to generate order information D1 including the request information. The HIS 6 transmits the order information D1 to the RIS 2 through the hospital network N1. The order information D1 includes patient information such as a name, ID, and sexuality of a patient to be photographed, and testing information indicating a testing condition such a testing ID for identifying the testing ordered by the doctor, an examined region, a photographing direction, and a body posture.

The RIS (Radiological Information System) 2 includes a computer which has a CPU, a storage section, an input section, a display section, and a communication section. The RIS 2 performs information management such as amedical appointment, a report on diagnoses, management of achievement, and management of material inventory in a radiological department in order to totally manage information in the radiological department system S. The RIS 2 receives the order information D1 transmitted from the HIS 6, determines a modality that takes photographs based on the order information D1, and transmits the order information D1 to the modality M. The RIS also transmits the order information D1 to the image testing apparatus 1 in addition to the modality M.

Examples of the modality M include a CT apparatus, a CR apparatus, a MRI apparatus, a mammographic apparatus, and an ultrasonic diagnostic apparatus. The modality M converts a data signal of an image which is taken in accordance with an operation by a photographic operator, into digital data which is compliant with a DICOM standard to generate medical image data D3. The modality M transmits the generated medical image data D3 to the image testing apparatus 1.

For example, the CR apparatus delivers radiation to a subject so that the radiation transmitted through the subject is absorbed by a plate-like photostimulable phosphor. The photostimulable phosphor is excited by laser light scanning, and the excitation energy of the radiation accumulated in the photostimulable phosphor is given off as light. Then, a data signal of a radiation image is obtained by photoelectric conversion, and the data signal is converted to digital signal to generate image data of the subject.

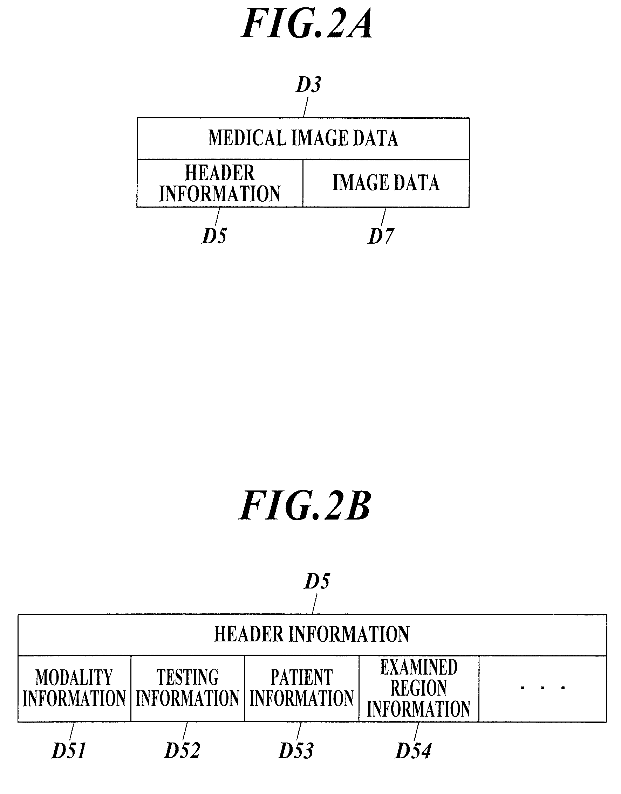

FIGS. 2A and 2B show examples of a data structure of medical image data D3. As shown in FIG. 2A, the medical image data D3 includes header information D5 and image data D7. As shown in FIG. 2B, the header information D5 includes modality information D51, testing information D52, patient information D53, and examined region information D54. The header information D5 is data which is compliant with a DICOM standard. In the header information D5 of FIG. 2B, main pieces of data in the embodiments are schematically shown while other pieces of data are omitted.

The modality information D51 is data indicating a type of modality which generates the image data D7 correlated with the header information D5. The modality information D51 is set by a corresponding modality M. The testing information D52 is data indicating contents of testing. The testing information D52 includes unique IDs assigned to each testing item ordered from the HIS 6 by a doctor, and a testing date and time.

The patient information D53 is data including a name, ID, and sexuality of the photographed patient. The examined region information D54 is data indicating an examined region of a patient, and is one of the testing conditions. The testing information D52, the patient information D53, and the examined region information D54 are set based on the order information D1 received from the RIS 2.

The image testing apparatus 1 receives the order information D1 transmitted from the RIS 2 and the medical image data D3 transmitted from the modality M, and accumulates data in an image testing waiting list table 72 to wait for image testing by a photographic operator. The "image testing" means an act of confirming the following items and correcting the medical image data D3 before the medical image data D3 is transmitted to PACS 5: whether or not photographing is carried out in accordance with the order information D1; whether or not the image data D7 has image quality suitable for diagnosis; and whether or not the order information D1 and the header information D5 are consistent with each other. After the image testing for the medical image data D3 by the photographic operator, the medical image data D3 is transmitted to the PACS 5 in accordance with the operation of the photographic operator.

The viewer 3 is a display device such as a liquid crystal display (LCD), and displays a medical image based on the medical image data D3. When the medical image data D3 to be displayed is designated by an interpreting doctor who interprets medical images, the viewer 3 retrieves the medical image data D3 from the PACS 5 and displays the medical image data D3.

The imager 4 is an image forming apparatus which includes a film digitizer. The imager 4 forms a medical image on a recording medium based on the medical image data D3 and outputs the medical image. When the medical image data D3 to be formed is designated by an interpreting doctor, the medical image data D3 is read out from the PACS 5, and the medical image is formed on the recording medium such as a heat sensitive film.

The PACS 5 is a database system which retains and manages the medical image data D3 transmitted from medical image generating apparatuses such as a CR apparatus, a CT apparatus, and a MRI apparatus to carry out a search and data analysis. The PACS 5 includes a CPU, a storage section, anda communication section. The PACS 5 stores the medical image data D3 in a relational database based on the header information D5 contained in the medical image data D3 received from the image testing apparatus 1. The PACS 5 carries out the search of the medical image data D3 using a search key such as a patient ID and a testing ID which are designated in accordance with operations by an interpreting doctor, and outputs the medical image data D3 to the viewer 3 and the imager 4.

### [First Embodiment]

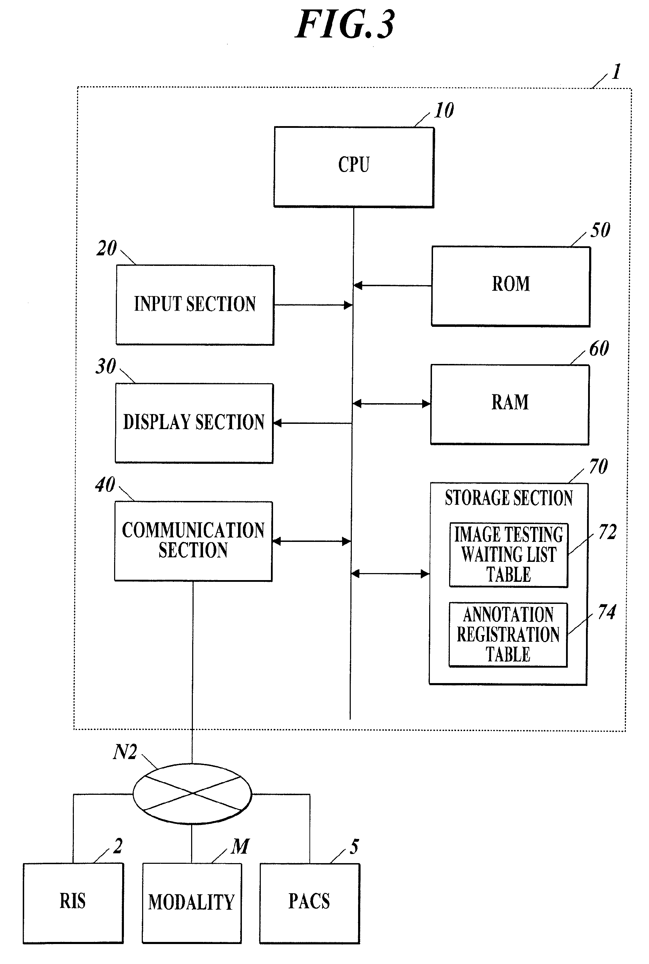

Next, a first embodiment of the image testing apparatus 1 will be described with reference to FIGS. 3 to 7B. First, a functional configuration of the image testing apparatus 1 in the first embodiment will be described.

FIG. 3 is a block diagram showing an example of a functional configuration of the image testing apparatus 1. As shown in FIG. 3, the image testing apparatus 1 includes a CPU 10, an input section 20, a display section 30, a communication section 40, a read only memory (ROM) 50, a random access memory (RAM) 60, and a storage section 70.

The CPU 10 is a control section which controls an operation of each functional section and data input and output between the functional sections to totally manage and control the image testing apparatus 1. Specifically, the CPU 10 reads out a program stored in the ROM 50 or the storage section 70 in accordance with a manipulated signal supplied from the input section 20, and executes the program. The CPU 10 updates a display screen of the display section 30, stores data in the storage section 70, and conducts data communication with an external device, in accordance with the processing result of the program.

The input section 20 includes various key groups such as a cursor key and a ten key and a pointing device such as a mouse and a touch panel. The input section 20 outputs a manipulated signal corresponding to a key operation by a user and a manipulated signal corresponding to a coordinate position on a screen designated by the pointing device, to the CPU 10.

The display section 30 includes a display such as a cathode-ray tube (CRT) or an LCD, and the display section 30 displays the screen and the medical image data D3 under the control of the CPU 10. The communication section 40 is a functional section which includes a LAN interface to conduct the data communication with the external device such as the RIS 2, the modality M, and the PACS 5 through the communication network N2.

The ROM 50 includes a nonvolatile semiconductor memory. The ROM 50 stores an initial program for various initial settings, hardware testing, andloadingofanecessaryprogram. The RAM 60 includes a rewritable semiconductor device. The RAM 60 temporarily stores various programs executed by the CPU 10 and data related to the programs.

The storage section 70 is a functional section which reads and writes data from and to a magnetic storage medium such as a hard disk drive (HDD) or an optical storage medium. As shown in FIG. 3, the image testing waiting list table 72 and an annotation registration table 74 are stored in the storage section 70.

The image testing waiting list table 72 is a data table in which the order information D1 transmitted from the RIS 2 and the medical image data D3 transmitted from the modality M are accumulated. The CPU 10 reads out the order information D1 and medical image data D3 stored in the image testing waiting list table 72 and display the order information D1 and medical image data D3 on the display section 30 in order display processing or image display processing.

The order display processing is executed when a photographic operator confirms consistency between the order information D1 and the header information D5 contained in the medical image data D3. In the order display processing, the CPU 10 reads out the order information D1 and the header information D5 of the medical image data D3, both of which contain identical testing information, from the image testing waiting list table 72. The CPU 10 displays the patient information and testing information contained in the order information D1 and the patient information and testing information contained in the header information D5 on the display section 30 in a manner associated with each other. When the patient information and testing information contained in the order information D1 and the patient information and testing information contained in the header information D5 are not consistent with each other, the photographic operator appropriately operates the input section 20 for correction.

The image display processing is executed when the photographic operator confirms a recording condition of the image data D7 contained in the medical image data D3. In the image display processing, the CPU 10 reads out the medical image data D3 from the image testing waiting list table 72, and displays contents of the header information D5 of the medical image data D3 and a medical image based on the image data D7 on the display section 30. If the photographic operator determines that the medical image displayed on the display section 30 is not suitable for diagnosis, the photographic operator operates the input section 20 so as to correct gradation (density/contrast) of the image data D7 and rotate or reverse the image data D7.

In the image display processing, annotation correction of the image data D7 is also executed. In the annotation correction processing, an image of an annotation (hereinafter referred to as "annotation image") recorded in the image data D7 by the modality M is corrected, or a new annotation image is added to the image data D7. The PU 10 refers to the annotation registration table 74 in the annotation correction processing.

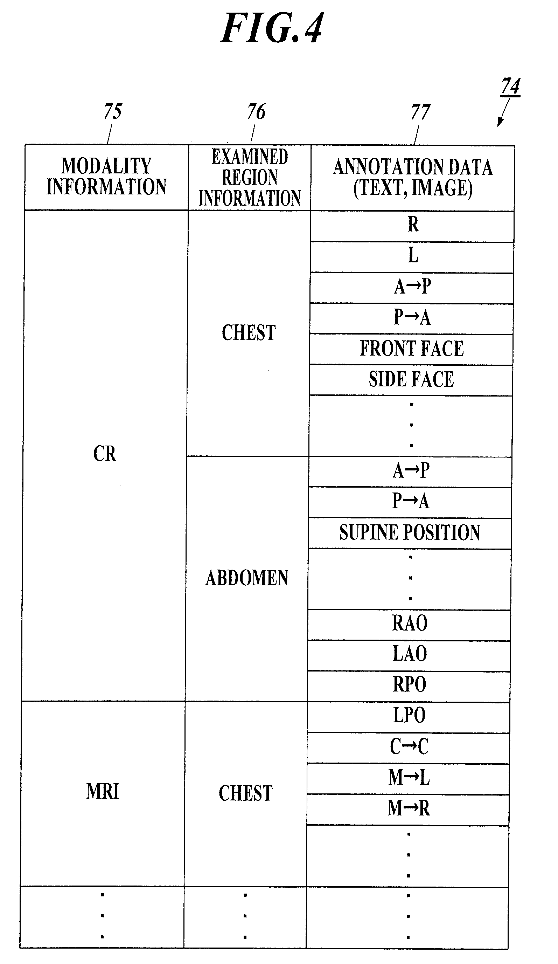

FIG. 4 shows an example of a data structure of the annotation registration table 74. As shown in FIG. 4, the annotation registration table 74 is a data table in which modality information 75, examined region information 76, and annotation data 77 are stored in a manner associated with each other. The modality information 75 is data indicating a type of modality. The examined region information 76 is data indicating an examined region of a patient.

The annotation data 77 is data indicating posture of a patient and a photographing direction when a region indicated by the examined region information 76 is photographed using a modality indicated by the associated modality information 75. The annotation data 77 includes text data and image data. The annotation data 77 is preset and stored in an initial state, and is appropriately set and registered by a user. Accordingly, the user can register the desired annotation data in advance 77 to customize annotation to be added or corrected with respect to each examined region for each modality.

For example, in FIG. 4, in connection with "CR" as the modality information 75, "chest" and "abdomen" as the examined region information 76 are stored as regions of a patient photographed by a "CR" apparatus. A plurality of pieces of annotation data 77 such as "R", "L", "A→P", "P→A", "front face", and "side face" are associated with "chest" as the examined region information 76.

In the annotation correction processing, the CPU 10 extracts the modality information D51 and the examined region information D54 from the header information D5 of the displayed medical image. The CPU 10 reads out the annotation data 77 suitable for the extracted modality information D51 and examined region information D54 from the annotation registration table 74, and displays a list of pieces of text data of the annotation data 77 on the display section 30.

That is, the CPU 10 selects the modality information 75 which is identical to the modality information D51 of the header information D5 from the annotation registration table 74, and selects one or more pieces of examined region information 76 that are associated with the modality information 75. The CPU 10 then selects the examined region information 76 which is identical to the examined region information D54 of the header information D5 from the pieces of examined region information 76. The CPU 10 reads out the annotation data 77 which is associated with the examined region information 76, and displays the list of the pieces of text data of the annotation data 77 on the display section 30.

The CPU 10 pastes an image data of the annotation data 77 selected by an operation of the input section 20 by the photographic operator at a position on the designated medical image, and updates the image data D7 of the medical image data D3. Since one or more pieces of annotation data 77 can properly be displayed according to the modality M, which is used to photograph the medical image data D3, and the examined region, the photographic operator can easily select the desired annotation data 77.

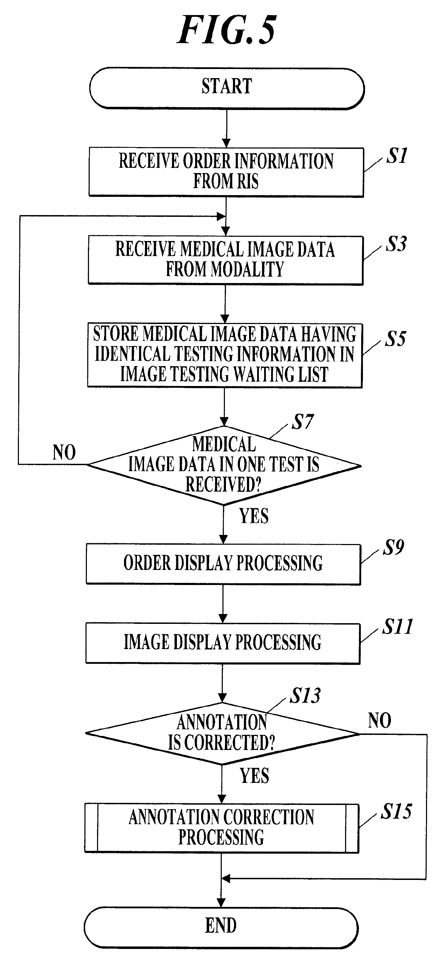

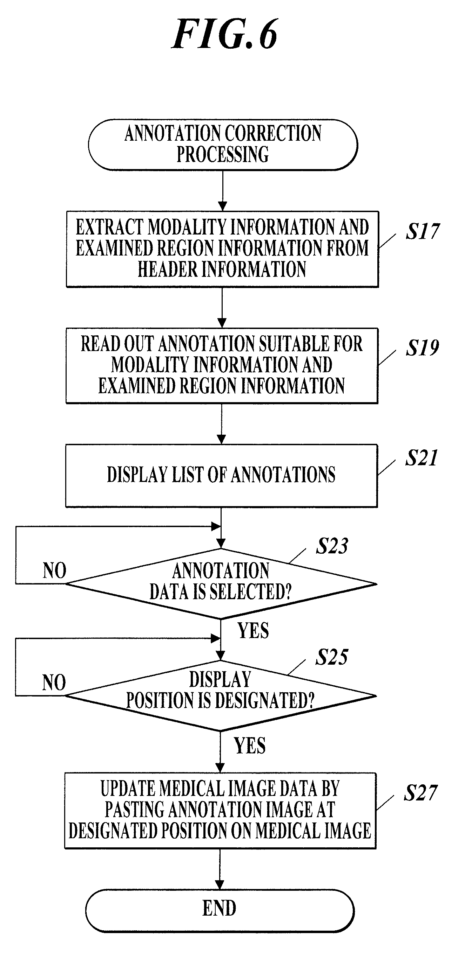

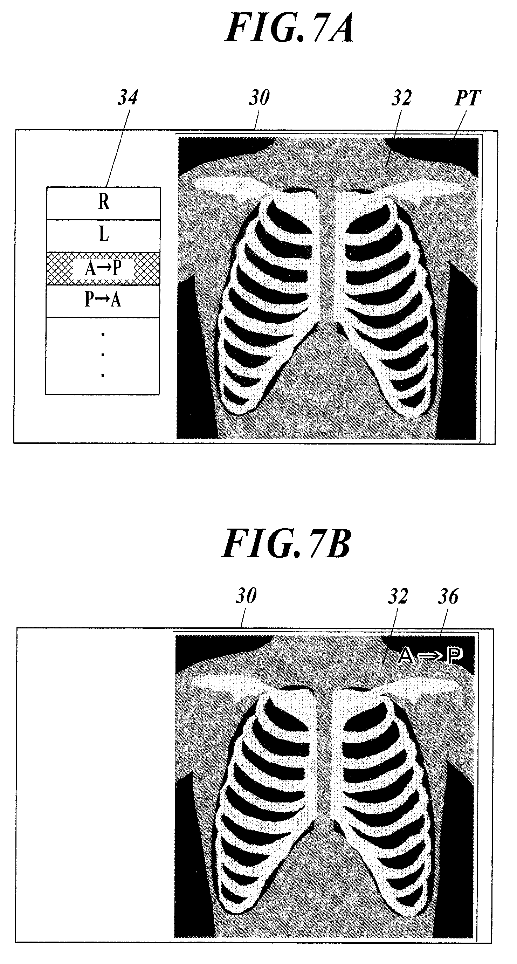

Next, a specific operationof the image testing apparatus 1 will be described with reference to flowcharts of FIGS. 5 and 6 and display examples on a screen of FIGS. 7A and 7B.

Upon receiving the order information D1 from the RIS 2, the CPU 10 stores the order information D1 in the image testing waiting list table 72 (Step S1). The CPU 10 then receives the medical image data D3 from the modality M (Step S3). The CPU 10 stores pieces of medical image data D3 which are classified into groups each having the same testing information D52, in the image testing waiting list table 72 (Step S5).

The CPU 10 determines whether or not medical image data D3 in one test is received (Step S7). If the CPU 10 determines that the medical image data D3 in one test is not received (NO in Step S7), the processing moves to Step S3 to wait for reception of the medical image data D3. A well known technique can appropriately be adopted to determine whether or not the medical image data D3 in one test is received. For example, the determination is made based on whether or not an end-of-testing signal is received from the modality M.

If the CPU 10 determines that the medical image data D3 in one test is received (YES in Step S7), the CPU 10 executes the order display processing and the image display processing (Steps S9 and S11). The CPU 10 then determines whether or not the annotation is corrected according to an operation of the input section 20 (Step S13). If the CPU 10 determines that the annotation is corrected (YES in Step S13), the CPU 10 executes annotation correction processing shown in FIG. 6 (Step S15). If the CPU 10 determines that the annotation is not corrected (NO in Step S13), the CPU 10 ends the processing of the flowchart shown in FIG. 5.

In the annotation correction processing shown in FIG. 6, the CPU 10 reads out the header information D5 of the medical image data D3 designated by an operation of the input section 20 by the user (photographic operator) from the image testing waiting list table 72, and extracts the modality information D51 and the examined region information D54 from the header information D5 (Step S17). At this time, the CPU 10 displays a medical image on the display section 30 on the basis of the image data D7 of the designated medical image data D3.

The CPU 10 reads out one or more pieces of annotation data 77 suitable for the extracted modality information D51 and examined region information D54 from the annotation registration table 74 as described above (Step S19), and displays a list of the pieces of text data of the annotation data 77 on the display section 30 (Step S21). The CPU 10 may display a list of annotation images based on the pieces of image data of the annotation data 77.

For example, where the modality information D51 and the examined region information D54 of the header information D5 contained in the designated medical image data D3 indicate "CR", "chest", respectively, the CPU 10 reads out the pieces of annotation data 77 such as "R", "L", "A→P", and "P→A", and displays a list 34 on the display section 30 along with a medical image 32 on the basis of the image data D7 as shown in FIG. 7A.

The CPU 10 waits until the annotation data 77 is selected from the list by an operation of the input section 20 by the user (Step S23) and the coordinate position at which an image of the selected annotation data 77 is displayed (that is, the annotation display position) is designated (Step S25). The CPU 10 synthesizes the medical image data D3 and the image data D7 to record the annotation image on the medical image so that the image of the selected annotation data 77 is pasted at the coordinate position designated on the medical image displayed on the display section 30 (Step S27), and the CPU 10 ends the annotation correction processing.

For example, when the annotation data 77 of "A→P" is selected from the list 34 displayed on the display section 30 using the cursor key or the pointing device as shown in FIG. 7A, and a coordinate point PT on the display section 30 is designated, an annotation image 36 is written so as to be pasted on the medical image 32 on the basis of the image data of the selected annotation data 77 as shown in FIG. 7B.

As described above, according to the first embodiment, the modality information D51 and the examined region information D54 are extracted from the header information D5 contained in the medical image data D3, and one or more pieces of annotation data 77 suitable for the modality information D51 and the examined region information D54 are read out from the annotation registration table 74 to display the list of the pieces of annotation data 77. Therefore, even if a plurality of kinds of modality are connected to the image testing apparatus 1, a proper annotation can be selected for each modality to record the annotation image on the medical image. A proper annotation for each examined region photographed by the modality M can also be selected and recorded on the medical image. Thus, the photographic operator can easily record the annotation image on the medical image without troublesome operations in which the annotation image is selected from a huge number of annotations.

If the annotation image has already recorded in the medical image data D3 transmitted from the modality M, the annotation image can be corrected by specifying a display position of the annotation on the recorded annotation image. Accordingly, it is possible to improve operability in adding or correcting an annotation and to realize the user-friendly medical image display apparatus 1.

In the first embodiment, the list of the pieces of annotation data 77 is displayed based on the examined region information D54 which is one of the testing conditions. Instead of the examined region information D54, the list of the pieces of annotation data 77 may be displayed based on another testing condition. For example, because the header information D5 which is compliant with the DICOM standard contains information on posture (posture information) in taking photographs, the list of the pieces of annotation data 77 can be displayed based on the posture information.

In such case, the storage section 70 stores an annotation registration table in which the modality information 75, the posture information, and the annotation data 77 are stored in a manner associated with each other, thereby executing the same processing as the first embodiment.

A plurality of different annotation registration tables 74 each storing pieces of annotation data 77 may be employed for different testing conditions in order to select one of the annotation registration tables according to one testing condition desired by a user (photographic operator). In such case, prior to Step S17 of FIG. 6, a testing condition is selected by an operation of the input section 20 by a user, and then the selected testing condition is extracted from the header information D5 in Step S17. In step S19, one or more pieces of annotation data 77 are read out from one of the annotation registration tables 74 which are classified according to the testing conditions.

Accordingly, whendisplaying the list of the annotations, the photographic operator can select one or more of the annotations which are classified according to the desired testing conditions such as an examined region, posture, and a photographing direction. Therefore, it is possible to realize the user-friendly image testing apparatus 1.

### [Second Embodiment]

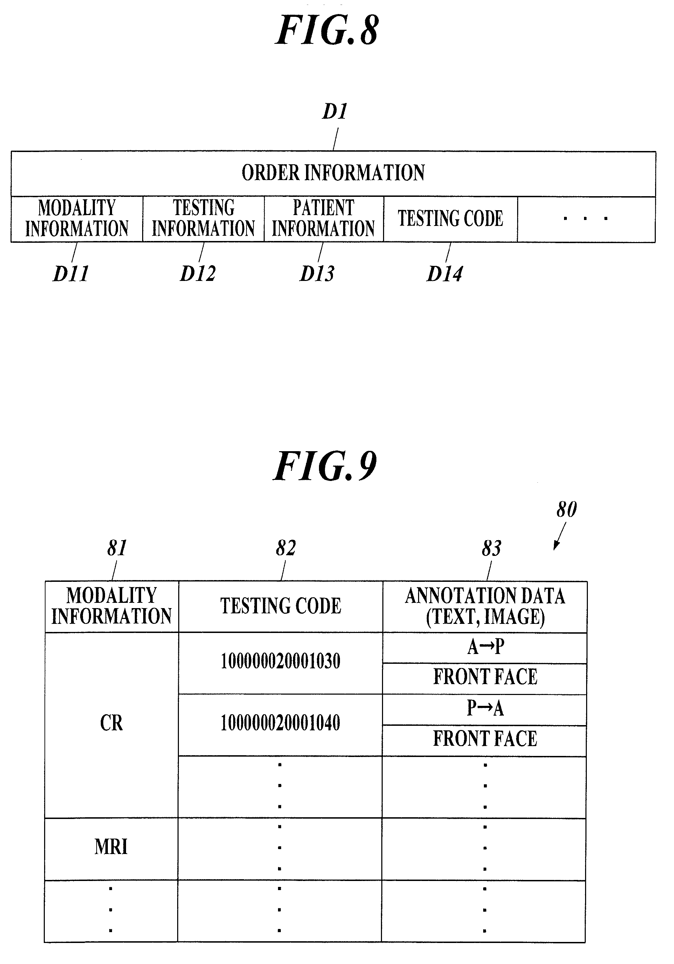

A second embodiment of the image testing apparatus 1 will be described with reference to FIGS. 8 to 10. In the image testing apparatus 1 of the second embodiment, the annotation registration table 74 shown in FIG. 4 is replaced with an annotation registration table 80 shown in FIG. 9. Because other functional configurations of the image testing apparatus 1 of the second embodiment are identical to those of the image testing apparatus 1 shown in FIG. 3, the detailed description will be omitted.

In the first embodiment, the list of the annotations is displayed based on the modality information D51 and examined region information D54 which are contained in the header information D5 of the medical image data D3 received from the modality M. In the second embodiment, the list of the annotations is displayed based on the order information D1 received from the RIS 2.

FIG. 8 shows an example of a data structure of the order information D1. As shown in FIG. 8, the order information D1 includes modality information D11, testing information D12, patient information D13, and testing code D14. For example, the order information D1 is compliant with HL7 of medical care data exchange standard. In the order information D1 of FIG. 8, main pieces of data in the second embodiment are schematically shown while other pieces of data are omitted.

The modality information D11 is data indicating a type of a modality M which takes photographs following orders from a doctor. The testing information D12 is data indicating contents of testing. The testing information D12 includes unique IDs assigned to each testing, and a testing date and time. The patient information D13 is data including a name, ID, and sexuality of a patient to be photographed. The testing code D14 is data indicating a region, a photographing direction, and posture of a patient, which are photographic subjects. The testing code D14 is described using a JJ1017-32 code. The order information D1 including the above-described pieces of data is set by the HIS 6 or the RIS 2.

FIG. 9 shows an example of a data structure of an annotation registration table 80 of the second embodiment. As shown in FIG. 9, the annotation registration table 80 is a data table in which modality information 81, a testing code 82, and annotation data 83 are stored in a manner associated with each other. The modality information 81 is data indicating a type of modality. The testing code 82 is data indicating a region which is a photographic subject. The testing code 82 is described in the same code format as the testing code D14 of the order information D1.

The annotation data 83 is data indicating posture and a photographing direction of a patient corresponding to the testing code 82 in the modality M of the corresponding modality information 81. The annotation data 83 includes text data and image data. The annotation data 83 may be preset and stored in the initial state or appropriately set by a user.

For example, in FIG. 9, a plurality of testing codes 82 such as "100000020001030" and "100000020001040" are associated with "CR" of the modality information 81. For example, the testing code 82 of "100000020001030" means that an examined region is a chest and the region is photographed from a front face (A→P). The testing code 82 of "100000020001030" is associated with the plurality of pieces of annotation data 83 such as "A→P" and "front face".

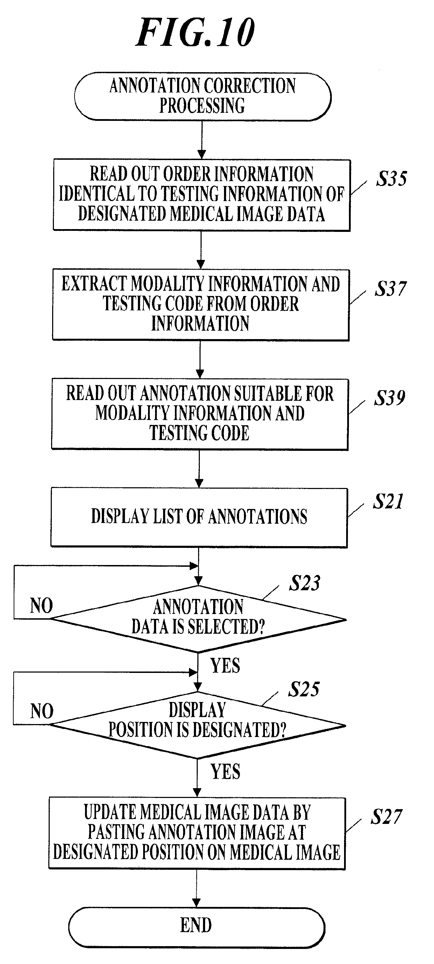

FIG. 10 is a flowchart for explaining annotation correction processing in the second embodiment. In Fig. 10, the same reference numerals are given without adding explanations for those steps that are the same as the annotation correction processing in the first embodiment of FIG. 6.

As shown in FIG. 10, the CPU 10 reads out the header information D5 of the medical image data D3 designated by an operation of the input section 20 by a user (photographic operator) from the image testing waiting list table 72. The CPU 10 reads out the order information D1 including the same testing information D12 as the testing information D52 in the header information D5 from the image testing waiting list table 72 (Step S35). At this time, a medical image is displayed on the display section 30 on the basis of the image data D7 of the designated medical image data D3.

The CPU 10 extracts the modality information D11 and the testing code D14 from the order information D1 (Step S37) . The CPU 10 reads out one or more pieces of annotation data 83 suitable for the extracted modality information D11 and testing code D14 from the annotation registration table 80 (Step S39).

That is, the CPU 10 selects the modality information 81 that is the same as the modality information D11 of the order information D1 from the annotation registration table 80, and further selects one ormore testing codes D14 associated with the modality information 81. Then, the CPU 10 selects one of the testing codes 82 that is the same as the testing code D14 of the order information D1, and reads out the annotation data 83 associated with the testing code 82.

As with the first embodiment, the CPU 10 displays the list of pieces of text data of the annotation data 83 which are read out in Step S39 on the display section 30. The CPU 10 pastes the selected annotation data 83 at the coordinate position designated on the medical image, and updates the image data D7 of the medical image data D3 (Steps S21 to S27).

As described above, according to the second embodiment, the modality information D11 and the testing code D14, which are contained in the order information D1 received from the RIS 2, are extracted, and the pieces of annotation data 83 suitable for the modality information D11 and the testing code D14 are read out from the annotation registration table 80 to display the list of the pieces of annotation data 83. Because the testing code D14 described in a code format is detailed data of a region, a photographing direction, and posture in taking photographs, it is possible to display the list of the pieces of annotation data 83 even more specific based on the testing conditions in taking photographs.

Therefore, the photographic operator can easily select an annotation without a troublesome operation that the photographic operator has to select one of a huge number of annotations. Accordingly, operability in adding or correcting the annotation can be improved to realize the user-friendly image testing apparatus 1.

The above embodiments are examples of the application of the present invention, and the present invention is not limited to the above-described embodiments. For example, both the annotation registration tables 74 and 80 of the first and second embodiments are stored in the storage section 70. In such case, the list of the pieces of annotation data may be displayed based on the modality information D51 and the examined region information D54 which are contained in the medical image data D3, and on the modality information D11 and the testing code D14 which are contained in the order information D1.

### Industrial Applicability

The present invention can be utilized in a medical field. The present invention can be applied to an image testing apparatus that receives order information transmitted from a HIS or a RIS and medical image data transmitted from a modality, and display the order information and the medical image data so that a photographic operator can visually check the order information and the medical image data to correct and adjust the data.

### Description of Reference Numerals

- 1: image testing apparatus
- 10: CPU
- 20: input section
- 30: display section
- 40: communication section
- 70: storage section
- 72: image testing waiting list table
- 74: annotation registration table
- 75: modality information
- 76: examined region information
- 77: annotation data
- D1: order information
- D11: modality information
- D12: testing information
- D14: testing code
- D3: medical image data
- D5: header information
- D51: modality information
- D52: testing information
- D54: examined region information
- D7: image data
- M: modality

## Claims

1. A medical image display apparatus comprising:
a storage section to store annotation data in a manner associated with a medical image generating apparatus for each medical image generating apparatus;
a display section to display a medical image based on medical image data received from the medical image generating apparatus;
a list display section to read out annotation data from the storage section, the annotation data being associated with the medical image generating apparatus from which the medical image data is received, and to display a list of the annotation data;
a selection section to select annotation data from the list displayed by the list display section in accordance with an operation by a user; and
a synthesizing section to synthesize the received medical image data and the annotation data selected by the selection section.

2. The medical image display apparatus of claim 1, further comprising an obtaining section to obtain a testing condition in the medical image generating apparatus, wherein
the storage section includes a testing condition storage section to store annotation data in a manner associated with each testing condition, and
the list display section reads out annotation data from the testing condition storage section, the annotation data being associated with the testing condition obtained by the obtaining section, and displays a list of the annotation data.

3. A medical image display apparatus comprising:
a testing condition storage section to store annotation data in a manner associated with a testing condition in a medical image generating apparatus for each testing condition;
a display section to display a medical image based on medical image data received from the medical image generating apparatus;
an obtaining section to obtain a testing condition in the medical image generating apparatus from which the medical image data is received;
a list display section to read out annotation data from the testing condition storage section, the annotation data being associated with the testing condition obtained by the obtaining section, and to display a list of the annotation data;
a selection section to select annotation data from the list displayed by the list display section in accordance with an operation by a user; and
a synthesizing section to synthesize the received medical image data and the annotation data selected by the selection section.

4. The medical image display apparatus of either claim 2 or 3, wherein the medical image data contains a testing condition in the medical image generating apparatus,
the obtaining section includes an extraction section to extract the testing condition from the received medical image data, and
the list display section reads out annotation data from the testing condition storage section, the annotation data being associated with the testing condition extracted by the extraction section, and displays a list of the annotation data.

5. The medical image display apparatus of any one of claims 2 to 4, wherein the obtaining section includes a reception section to receive a testing condition in the medical image generating apparatus from a medical information system which orders generation of the medical image data, and
the list display section reads out annotation data from the testing condition storage section, the annotation data being associated with the testing condition received by the reception section, and displays a list of the annotation data.

6. The medical image display apparatus of any one of claims 1 to 5, further comprising a designation section to designate an annotation display position on the medical image displayed by the display section, wherein
the synthesizing section synthesizes the annotation data selected by the selection section at the position designated by the designation section on the medical image, and updates the medical image data.

7. A program causing a computer to function as:
a storage section to store annotation data in a manner associated with a medical image generating apparatus for each medical image generating apparatus;
a display section to display a medical image based on medical image data received from the medical image generating apparatus;
a list display section to read out annotation data from the storage section, the annotation data being associated with the medical image generating apparatus from which the medical image data is received, and to display a list of the annotation data;
a selection section to select annotation data from the list displayed by the list display section in accordance with an operation by a user; and
a synthesizing section to synthesize the received medical image data and the annotation data selected by the selection section.

8. A program causing a computer to function as:
a testing condition storage section to store annotation data in a manner associated with a testing condition in a medical image generating apparatus for each testing condition;
a display section to display a medical image based on medical image data received from the medical image generating apparatus;
an obtaining section to obtain a testing condition in the medical image generating apparatus from which the medical image data is received;
a list display section to read out annotation data from the testing condition storage section, the annotation data being associated with the testing condition obtained by the obtaining section, and to display a list of the annotation data;
a selection section to select annotation data from the list displayed by the list display section in accordance with an operation by a user; and
a synthesizing section to synthesize the received medical image data and the annotation data selected by the selection section.
